(19) Europäisches Patentamt / European Patent Office / Office européen des brevets

(11) **EP 2 119 443 B1**

(12) **EUROPEAN PATENT SPECIFICATION**

(45) Date of publication and mention
of the grant of the patent:
**20.08.2014 Bulletin 2014/34**

(21) Application number: **08703408.8**

(22) Date of filing: **17.01.2008**

(51) Int Cl.:
*A61K 31/4365* (2006.01)     *A61K 31/202* (2006.01)
*A61P 3/00* (2006.01)     *A61P 3/06* (2006.01)
*A61P 3/10* (2006.01)     *A61P 7/02* (2006.01)
*A61P 9/00* (2006.01)     *A61P 9/10* (2006.01)
*A61P 41/00* (2006.01)     *A61P 43/00* (2006.01)

(86) International application number:
**PCT/JP2008/050554**

(87) International publication number:
**WO 2008/088030 (24.07.2008 Gazette 2008/30)**

(54) **COMPOSITION FOR PREVENTION OR TREATMENT OF DISEASE ASSOCIATED WITH THROMBUS OR EMBOLUS**

ZUSAMMENSETZUNG ZUR PRÄVENTION ODER BEHANDLUNG VON ERKRANKUNGEN IM ZUSAMMENHANG MIT EINEM THROMBUS ODER EMBOLUS

COMPOSITION DESTINÉE À LA PRÉVENTION OU AU TRAITEMENT D'UNE MALADIE ASSOCIÉE À UN THROMBUS OU À UN EMBOLE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **17.01.2007 JP 2007008590**

(43) Date of publication of application:
**18.11.2009 Bulletin 2009/47**

(73) Proprietor: **MOCHIDA PHARMACEUTICAL CO., LTD.**
**Shinjuku-ku**
**Tokyo 160-8515 (JP)**

(72) Inventors:
• **KAWANO, Hiroyuki**
**Shinjuku-ku**
**Tokyo 160-8515 (JP)**
• **SAITO, Ryo**
**Shinjuku-ku**
**Tokyo 160-8515 (JP)**

(74) Representative: **Grünecker, Kinkeldey, Stockmair & Schwanhäusser**
**Leopoldstrasse 4**
**80802 München (DE)**

(56) References cited:
**WO-A2-2007/098020**

• **"The Merck Manual of Diagnosis and Therapy - seventeenth edition" 1999, Merck Research Laboratories , XP002564618 , pages 1417-1424 * the whole document ***
• **SAITO R ET AL: "Effects of ethyl eicosapentaenoate (EPA-E), clopidogrel and their combination on platelet aggregation and bleeding time" JAPANESE PHARMACOLOGY AND THERAPEUTICS 2007 JP, vol. 35, no. 2, 2007, pages 179-185, XP009128292 ISSN: 0386-3603**
• **GEIGER J. ET AL.: 'Specific Impairment of Human Platelet P2YAC ADP Receptor-Mediated Signaling by the Antiplatelet Drug Clopidogrel' ARTERIOSCELEROSIS, THROMBOSIS, AND VASCULAR BIOLOGY vol. 19, 1999, pages 2007 - 2011, XP001221745**
• **'Iyakuhin Interview Form "Ko Kesshobanzai Plavix"' June 2006, pages 1 - 60, XP008119401**
• **SATO M. ET AL.: 'Effects of Highly Purified Ethyl All-cis-5,8-11,14,17-icosapentaenoate (EPA-E) on Rabbit Platelets' BIOLOGICAL & PHARMACEUTICAL BULLETIN vol. 16, no. 4, 1993, pages 362 - 367, XP008116207**

(Cont. next page)

• IKEDA Y.: 'Junkankiyaku no Tsukaikata 2000 Sonoto no Junkankiyaku no Tsukaikata Ko Kesshoban'yaku' MEDICINA vol. 37, no. 8, 2000, pages 1325 - 1328, XP008116828

• YANAGISAWA K. ET AL.: 'Ethyl Icosapentate to Ticlopidine no Heiyo Toyo ni Okeru Shukketsu Jikan to Kesshoban Gyoshuno ni Oyobosu Eikyo' CLINICAL REPORT vol. 26, no. 7, 1992, pages 3193 - 3198, XP008116777

**Description**

TECHNICAL FIELD

The present invention relates to compositions for preventing or treating diseases associated with thrombi or emboli and, particularly, compositions adapted for a combined application of at least one selected from the group consisting of clopidogrel and its pharmaceutically acceptable salts and at least one selected from the group consisting of icosapentaenoic acid as well as its pharmaceutically acceptable salts and esters.

BACKGROUND ART

The inner surface of a blood vessel is covered with vascular endothelial cells, which effect an inhibitory mechanism against platelet adhesion and aggregation. Under angiopathy, however, the collagen in the intravascular hypodermis is exposed. Von Willebrand factor (vWF) in the blood is activated when bound to the exposed collagen, to have glycoprotein (GP) Ib on the platelet membrane bound thereto, leading to the adhesion and aggregation of platelets. Collagen receptors on the platelet membrane bind directly to the collagen and are also contributory to the platelet adhesion and aggregation. Moreover, destructed cells release ADP, and the release of coagulation factor-activating substances brings about formation of thrombin. Such factors activate platelets intensely. Collagen, as being exposed when a vessel wall is damaged, can be considered as the most physiological platelet-activating substance which induces platelet adhesion and aggregation (Non-Patent Document 1). As stated above, collagen is exposed if vascular endothelial cells or a vessel wall is damaged for some reason, and then, due to the platelet aggregation caused by the collagen, thrombus formation is induced.

Vascular endothelial cells are those cells which are programmed severalfold to be nonthrombogenic. As a matter of course, exfoliation of vascular endothelial cells leads to a localized thrombus formation. Thrombi will also be generated with the endothelial cells not exfoliated but functionally or organically damaged. If the endothelial cells are damaged in themselves, or their functions are injured, thrombi are generated in the blood vessel. Major examples of the factor of such vascular endothelial cell damage include glycated proteins (or AGEs) generated in the blood vessel of a subject with diabetes, oxidized or degenerated LDL, and endotoxins. Also known as a vascular endothelial cell-damaging factor are mechanical factors such as the shearing stress due to pressure or flow, and biochemical factors such as thrombin, inflammatory cytokines (e.g., IL-1.or TNF-$\alpha$), and free radicals (Non-Patent Document 2). Under a pathological condition in which a vascular endothelial cell-damaging factor is activated, or on which the factor is exerting influence, vascular endothelial cells are damaged and collagen is exposed, which makes the collagen-induced aggregation of platelets liable to occur.

In many countries, such drugs as ticlopidine, clopidogrel sulfate (hereafter also referred to as clopidogrel), aspirin, and cilostazol are clinically used as an antiplatelet agent, used for the prevention and treatment of ischemic heart diseases, cerebral infarction, and the like caused by thrombi or emboli.

Polyunsaturated fatty acids are known as an example of the compounds having an ameliorative effect on hyperlipidemia. The polyunsaturated fatty acids are defined as fatty acids each having two or more carbon-carbon double bonds in a molecule, and are classified in accordance with the positions of double bonds into $\omega$-3 fatty acids, $\omega$-6 fatty acids, and so forth. The $\omega$-3 polyunsaturated fatty acids include $\alpha$-linolenic acid, icosapentaenoic acid (or EPA), and docosahexaenoic acid (DHA), and the $\omega$-6 polyunsaturated fatty acids include linoleic acid, $\gamma$-linolenic acid, and arachidonic acid. The polyunsaturated fatty acids are derived from natural products, and incorporated in various foods or sold as a health food or medicament because of their having diverse actions including antiarteriosclerotic action, platelet aggregation-suppressing action, hypolipidemic action, antiinflammatory action, antitumor action and central action, as well as high safety.

Recently in Japan, a large-scale, clinical intervention study, the Japan EPA Lipid Intervention Study (JELIS) by title, was conducted for the first time in the world with respect to coronary events by using a formulation containing eicosapentaenoic acid (EPA) of high purity, and it is reported that administering ethyl ester of a high-purity EPA (EPA-E) in addition to a statin drug significantly suppressed coronary events as compared with the administration of the statin drug alone. The EPA-E is used widely in the clinical scene in order to ameliorate ulcers, pain and cryesthesia associated with arteriosclerosis obliterans, or as a therapeutic agent against hyperlipidemia. The actions of the ester such as improvement in lipid metabolism, amelioration of damage to the vascular endothelium, plaque stabilization, and inhibition of thrombus formation by suppressing platelet aggregation as well, are elucidated in mechanism.

On the other hand, the active metabolite produced by the metabolization of clopidogrel sulfate in the liver has an antiplatelet action based on the antagonism to ADP receptors on a platelet, and clopidogrel sulfate has been approved in Japan in recent years for application as indicated for the suppression of recurrence of ischemic cerebrovascular accidents (except for cardiogenic cerebral embolism). The guideline jointly produced by the American College of Cardiology, the American Heart Association, and the Society for Cardiovascular Angiography and Interventions recommends

3

to administer clopidogrel to a patient having undergone percutaneous coronary angioplasty. Clopidogrel sulfate may be applied in combination with aspirin.

Interaction between the drugs applied in combination varies with the nature of individual drugs, and it is stated in the Drug Interview Form of a clopidogrel formulation that care should be given to the interaction with other drugs which increase a risk of bleeding, as well as that the formulation should be administered with care to the patients suffering from sustained hypertension (Non-Patent Document 3). In addition, application of drugs in combination may contrarily make such side effects as platelet aggregation and bleeding severer, so that it is general in the medical scene to avoid drug combination unless the effectiveness and safety of the combination is ensured, even if it seems to be favorable. Yanagisawa et al, vol. 26, nat, 1992, pages 3193-3198 discloses the combination of ticlopidine, and ethyl eicosapentaenoate for use in treating diseases associated with platelet aggregation.

> Non-Patent Document 1: Yukio OZAKI, "Saishin Igaku (current medicine)," No. 2, Vol. 55; pp. 41-51.
> Non-Patent Document 2: Ikuro MARUYAMA, "Kekkan To Naihi (blood vessels and endothelia)," No. 6, Vol. 9 (1999); pp. 136-140.
> Non-Patent Document 3: Drug Interview Form of the antiplatelet agent, Plavix, June, 2006 (2nd Edition); pp. 43 and 44.

DISCLOSURE OF THE INVENTION

PROBLEMS TO BE SOLVED BY THE INVENTION

It is an object of the present invention to provide a composition for preventing or treating a thrombus- or embolus-associated disease, which is not only excellent in safety and effectiveness but easy to use when applied against the thrombus- or embolus-associated disease, in particular that with platelet activation or aggregation, with damage to the vascular endothelium, or that involving a risk of bleeding.

MEANS TO SOLVE THE PROBLEMS

The object is achieved by finding that clopidogrel sulfate having the antagonism to ADP receptors and ethyl icosapentate (hereafter also referred to as EPA-E) applied in combination have an effect of synergistically suppressing the platelet aggregation induced by collagen. Moreover, there were no side effects such as elongation of the bleeding time, which reveals a high safety of the application of the two drugs in combination.

The present invention provides the following.

(1) A composition for use in the prevention or treatment of a thrombus- or embolus-associated disease, comprising, as active ingredients to be applied in combination, at least one selected from the group consisting of clopidogrel and its pharmaceutically acceptable salts and at least one selected from the group consisting of icosapentaenoic acid as well as its pharmaceutically acceptable salts and esters, wherein the application in combination includes administering a formulation containing both active ingredients, and administering the two active ingredients as separate formulations simultaneously, or separately with a certain time lag.

(2) A composition for use in the prevention or treatment according to the above (1), wherein said composition is administered in a subject suffering from a disease or condition with vascular endothelial cell damage.

(3) The composition for use in the prevention or treatment according to the above (2), wherein the disease or condition with vascular endothelial cell damage is a disease or condition which includes at least one symptom selected from the group consisting of high glycated-protein (AGE) levels, diabetes, hyperglycemia, diabetic complications, arteriosclerosis obliterans (ASO) complicated with diabetes, high ox-LDL levels, hyperlipidemia involving high ox-LDL levels, and inflammation marker abnormality.

(4) The composition for use in the prevention or treatment according to the above (2) or (3), wherein the patient is suffering from a disease or condition whose amelioration requires suppression of platelet aggregation in spite of a risk of bleeding.

(5) The composition for use in the prevention or treatment according to any one of the above (2) through (4), wherein said composition is administered in a subject selected from the group consisting of a subject having undergone revascularization and a subject with history of stroke.

(6) The composition for use in the prevention or treatment according to any one of the above (1) through (5), wherein the thrombus- or embolus-associated disease comprises at least one selected from the group consisting of thrombosis, embolism, cardiovascular death, fatal myocardial infarction, sudden cardiac death, non-fatal myocardial infarction, recurrence of myocardial infarction, angina pectoris decubitus, unstable angina pectoris, transient ischemic attack, congestive heart failure, new onset of exertional angina pectoris, destabilization of angina pectoris, ischemic heart disease, postoperative restenosis after cardiac revascularization, cardiovascular events occurring in an unstable phase after cardiac revascularization, cardiovascular events in a patient beyond an unstable phase after cardiac revascularization, progression of atherosclerosis, stroke, recurrence of stroke in a patient with history of stroke, recurrence after an ischemic cerebrovascular accident, consciousness disorder or nervous symptoms caused by a cerebrovascular accident, cerebral infarction, transient cerebral ischemic attack, subarachnoid hemorrhage, cerebral thrombosis, cerebral embolism, lacunar infarct, syncope, ulcers, pain and cryesthesia associated with arteriosclerosis obliterans, or thrombotic and/or atherothrombotic events occurring in myocardial infarction, stroke, ischemic cerebrovascular accidents, peripheral arterial diseases, acute coronary syndrome and non-ST-elevation acute coronary syndrome (unstable angina pectoris or non-Q-wave myocardial infarction).

(7) The composition for use in the prevention or treatment according to any one of the above (1) through (6), comprising clopidogrel sulfate and ethyl icosapentate as active ingredients.

(8) The composition for prevention or treatment according to any one of the above (1) through (7), wherein the dose ratio of clopidogrel sulfate to ethyl icosapentate is 1:10 to 1:80.

(9) The composition for use in the prevention or treatment according to any one of the above (1) through (8), which is a composite formulation of clopidogrel sulfate and ethyl icosapentate.

(10) The composition for use in the prevention or treatment according to any one of the above (2) through (9), wherein said composition is applied as a platelet aggregation inhibitor.

(11) Use of at least one selected from the group consisting of clopidogrel and its pharmaceutically acceptable salts and at least one selected from the group consisting of icosapentaenoic acid as well as its pharmaceutically acceptable salts and esters for manufacturing a composition for the prevention or treatment of a thrombus- or embolus-associated disease or condition in a subject suffering from a disease or condition with vascular endothelial cell damage, to be applied in combination, wherein the application in combination includes administering a formulation containing both active ingredients, and administering the two active ingredients as separate formulations simultaneously, or separately with a certain time lag.

(12) At least one selected from the group consisting of icosapentaenoic acid as well as its pharmaceutically acceptable salts and esters for use in the prevention or treatment of a thrombus- or embolus-associated disease to be applied in combination with at least one selected from the group consisting of clopidogrel and its pharmaceutically acceptable salts simultaneously, or separately with a certain time lag.

(13) At least one selected from the group consisting of clopidogrel and its pharmaceutically acceptable salts for use in the prevention or treatment of a thrombus- or embolus-associated disease to be applied in combination with at least one selected from the group consisting of icosapentaenoic acid as well as its pharmaceutically acceptable salts and esters simultaneously, or separately with a certain time lag.

EFFECTS OF THE INVENTION

The combined application of at least one selected from the group consisting of clopidogrel and its pharmaceutically acceptable salts and at least one selected from the group consisting of icosapentaenoic acid as well as its pharmaceutically acceptable salts and esters realizes an intense platelet aggregation-suppressing action and, accordingly, is effective for the prevention or treatment of thrombus- or embolus-associated diseases. It is suitably employed against, for instance, diseases or conditions with platelet activation or aggregation, in particular conditions with platelet activation or aggregation by collagen, or conditions with damage to the vascular endothelium. The combined application as above least brings about bleeding as a side effect, and is safely employed even against diseases involving a risk of bleeding.

BRIEF DESCRIPTION OF THE DRAWINGS

[FIG. 1] FIG. 1 is a graph showing effects on the platelet aggregation induced by ADP.
[FIG. 2] FIG. 2 is a graph showing effects on the platelet aggregation induced by collagen.
[FIG. 3] FIG. 3(A) is a graph showing effects on a coagulation-related parameter, PT, and FIG. 3(B) is a graph showing effects on a coagulation-related parameter, APTT.
[FIG. 4] FIG. 4 is a graph showing effects on the bleeding time.

BEST MODE FOR CARRYING OUT THE INVENTION

The following is a detailed description of the present invention.
The present invention provides a composition for the prevention or treatment of a thrombus- or embolus-associated disease, including, as active ingredients to be applied in combination, at least one selected from the group consisting of clopidogrel and its pharmaceutically acceptable salts and at least one selected from the group consisting of icosapentaenoic acid as well as its pharmaceutically acceptable salts and esters. The composition of the present invention is a combined composition in which to be applied in combination at least one selected from the group consisting of clopidogrel and its pharmaceutically acceptable salts and at least one selected from the group consisting of icosapentaenoic acid as well as its pharmaceutically acceptable salts and esters are used in combination as active ingredients.

<Clopidogrel sulfate>

In the present invention, clopidogrel may be used in the form of a pharmaceutically acceptable salt thereof, with clopidogrel sulfate being preferable.

Clopidogrel sulfate is represented by the structural formula as below, and its chemical name is (+)-(s)-methyl 2-(2-chlorophenyl)-2-(4,5,6,7-tetrahydrothieno[3,2-c]pyridin-5-yl) acetate monosulfate (IUPAC). This compound is commercially available from Daiichi Pharmaceutical Co., Ltd. under the trade name Plavix™. In foreign countries, it is marketed by Bristol-Myers Squibb Company and sanofi-aventis under the trade name Plavix™/Iscover™.

<Ethyl icosapentate>

The term "icosapentaenoic acid" used herein refers to all-cis-5,8,11,14,17-icosapentaenoic acid. The "icosapentaenoic acid as well as its pharmaceutically acceptable salts and esters" include ethyl ester and other alkyl esters as well as such esters as glyceride. Icosapentaenoic acid can react with an inorganic base to produce sodium salt, potassium salt, or the like, with an organic base to produce benzylamine salt, diethylamine salt, or the like, and with a basic amino acid to produce arginine salt, lysine salt, or the like. Unless otherwise specified, EPA in the present invention includes salts and esters as above. The preferred is ethyl icosapentate. Many kinds of EPA-containing fatty acids are known that contain EPA as an active ingredient and allow the present invention to have the expected effects. The percentage of EPA to all the fatty acids contained, and the EPA dose as well, do not need to be specified critically, but EPA is preferably of high purity. If EPA-E is used as an active ingredient, for instance, the percentage of EPA-E to all the fatty acids and their derivatives is preferably not less than 40% by weight, more preferably not less than 90% by weight, and even more preferably not less than 96.5% by weight. The daily dose of EPA-E is, for instance, 0.3 to 6 g/day, preferably 0.9 to 3.6 g/day, and more preferably 1.8 to 2.7 g/day.

EPA used in the composition of the present invention involves little such impurities as saturated fatty acids and arachidonic acid, which unfavorably affect cardiovascular events, as compared with fish oils or concentrates thereof, and can act effectively without a problem of overnutrition or excess intake of vitamin A. In addition, because of its ester form, EPA has a higher oxidation stability than fish oils and the like chiefly in trigrycelide form, so that a composition containing EPA can be made to be fully stable by adding a conventional antioxidant.

Examples of other preferable fatty acids to be contained include ω-3 long-chain unsaturated fatty acids, particularly DHA and DHA-E. If DHA-E is used, for instance, the composition ratio of EPA-E to DHA-E, the percentage of EPA-E plus DHA-E to all the fatty acids contained, and the dose of EPA-E plus DHA-E do not need to be specified critically, while the composition ratio EPA-E/DHA-E is preferably not less than 0.8, more preferably not less than 1.0, and even more preferably not less than 1.2. EPA-E plus DHA-E are preferably contained at so high a purity that, for instance, the percentage of EPA-E plus DHA-E to all the fatty acids and derivatives thereof is not less than 40% by weight, more preferably not less than 80% by weight, especially not less than 90% by weight. The daily dose of EPA-E plus DHA-E is, for instance, 0.3 to 10 g/day, preferably 0.5 to 6 g/day, and more preferably 1. to 4 g/day. It is preferable that the content of a long-chain saturated fatty acid which may additionally be contained is low, and the content of a long-chain unsaturated fatty acid of ω-6 type, particularly arachidonic acid, is also low, with the content of less than 2% by weight, or even less than 1% by weight, being preferred.

Soft capsules containing EPA-E of high purity, EPADEL™ and EPADEL S™, have already been marketed in Japan as therapeutic agents against arteriosclerosis obliterans and hyperlipidemia which are safe with reduced side effects, and in these capsules, the percentage of EPA-E to all the fatty acids contained is 96.5% by weight or more. Soft capsules containing ca. 46% by weight EPA-E and ca. 38% by weight DHA-E (Omacor™ from Ross Products) are also commercially available in U.S.A. and other countries as a therapeutic agent against hypertriglyceridemia (diseases with high TG levels). Such agents may be purchased and used in the present invention.

In the present invention, "combined application of drugs" or "application of drugs in combination" includes application of a combination of drugs, that is to say, administering clopidogrel sulfate and ethyl icosapentate as a formulation containing both, and administering the two drugs as separate formulations simultaneously, or separately with a certain time lag, are included therein. In the mode of administration in which the two drugs are administered "as separate formulations simultaneously, or separately with a certain time lag," included are (1) administration of a composition containing clopidogrel sulfate as an active ingredient to a patient under treatment with ethyl icosapentate, and (2) administration of a composition containing ethyl icosapentate as an active ingredient to a patient under treatment with clopidogrel sulfate. Moreover, although the drugs "applied in combination" are not necessarily limited to concurrently existing in the body of a patient, in the blood for instance, "application of drugs in combination" is defined in the present invention as an application method in which one drug is administered while the actions or effects of the other drug are exerted in the body of a patient. Such an application method makes it possible to prevent or treat thrombus- or embolus-associated diseases effectively by using the composition of the present invention. With respect to this application method, it is preferable that the drugs applied in combination are concurrently present in the body of a patient, in the blood for instance, and that one drug is administered to a patient within 24 hours after the administration of the other drug.

In terms of the drugs of the invention, the mode of application in combination is not particularly limited as long as the drugs as active ingredients are combined with each other.

The following are exemplary modes: (1) The active ingredients are formulated at a time, and the single formulation thus obtained is administered. (2) The active ingredients are formulated separately, and the two formulations thus obtained are combined together into a kit or kept separate, and administered simultaneously from one and the same dosage route. (3) The active ingredients are formulated separately, and the two formulations thus obtained are combined together into a kit or kept separate, and administered separately with a certain time lag from one and the same dosage route. (4) The active ingredients are formulated separately, and the two formulations thus obtained are combined together into a kit or kept separate, and administered simultaneously from different dosage routes (from different sites of one and the same patient). (5) The active ingredients are formulated separately, and the two formulations thus obtained are combined together into a kit or kept separate, and administered separately with a certain time lag from different dosage routes (from different sites of one and the same patient).

In the case of separate administration with a certain time lag, clopidogral sulfate may be administered prior to ethyl icosapentate, or vice versa, for instance. In the case of simultaneous administration, the two drugs may or may not be mixed together immediately before administration if the dosage route is one and the same. It is also possible to administer the drugs at different periods by design for various purposes. Specifically, one drug may be administered and allowed to act when the effects of the other drug, which has previously been administered, begin to be developed or are being fully developed. The two drugs may be administered at a time so as to stop administration of one drug when the effects of both the drugs begin to be developed or are being fully developed. If the administration of a drug is stopped, the drug may gradually be reduced in dose. Administering one drug during the withdrawal of the other drug is also available.

<Indications of the inventive composition>

In the diseases or conditions for which the present invention is indicated, diseases or conditions with platelet activation or aggregation, in particular those with the platelet activation or aggregation by collagen, and diseases or conditions with vascular endothelial cell damage are included. Also included are diseases or conditions with the platelet activation or aggregation by ADP (adenosine 5'-diphosphate), and diseases or conditions involving a risk of bleeding.

The diseases for which the composition of the present invention is indicated are particularly those which are caused by thrombi or emboli, or involve them. For instance, thrombosis, embolism, or atherosclerosis is prevented from progression and/or treated effectively with the composition. In such diseases as above, not only arterial thrombosis but phlebothrombosis is included, and cerebral infarction due to auricular fibrillation is included as well. In this specification, "prevention of a disease" is defined as an action including one or more out of "reduction in the incidence rate of a disease," "retardation of the onset of a disease," and "suppression of the onset of a disease."

The composition of the present invention is suitable for the application against diseases or conditions with vascular endothelial cell damage. In a disease or condition with vascular endothelial cell damage, vascular endothelial cells are damaged for some reason to make collagen exposed, with platelet activation or aggregation being induced by the collagen. The composition of the present invention is desirably a composition to be applied to a patient suffering from a disease or condition with vascular endothelial cell damage.

Damage to vascular endothelial cells is caused by a mechanical factor such as surgical procedure and trauma, or a biochemical factor such as glycated protein (AGE), ox-LDL, endotoxin, thrombin, inflammatory cytokine (e.g., IL-1 or TNF-$\alpha$), and free radical (each factor being referred to in the present invention as a vascular endothelial cell-damaging factor). The composition of the present invention is suitable for the application against diseases or conditions involving such factors.

Examples of the disease or condition with the vascular endothelial cell damage due to a mechanical factor include conditions upon and/or after revascularization such as thrombolytic therapy, percutaneous transluminal coronary angioplasty, stenting, bypass operation, or artificial vessel transplantation, that is to say, vascular endothelial thickening, vascular restenosis, cardiovascular events occurring in the unstable phase after cardiac revascularization, and cardiovascular events in a patient beyond the unstable phase after cardiac revascularization, which are associated with the revascularization as above.

The term "cardiovascular events" used herein refers to the lesions on the cardiovascular system as a whole, including cardiovascular death (fatal myocardial infarction, sudden cardiac death), non-fatal myocardial infarction, cardiac revascularization (e.g., PTCA, PTCR, DCA, coronary stenting, A-C bypass, or the like), new onset of angina pectoris decubitus or exertional angina pectoris, and destabilization of angina pectoris (admission to a hospital, PTCA, PTCR, DCA, coronary stenting, A-C bypass, other cardiac-revascularization procedures, and so forth).

The unstable phase after revascularization is a postoperative phase lasting about three months in which vascular events due to the revascularization in itself are liable to occur. To treat and/or prevent a vascular event in a patient beyond the unstable phase after revascularization means to prevent the occurrence and/or recurrence of a vascular event after a lapse of six months following revascularization.

In the present invention, the procedure of revascularization is not particularly limited, and examples thereof include percutaneous transluminal coronary angioplasty (hereafter abbreviated as PTCA), percutaneous transluminal coronary recanalization (hereafter abbreviated as PTCR), directional coronary atherectomy (hereafter abbreviated as DCA), coronary stenting, and coronary bypass operation (hereafter abbreviated as A-C bypass).

The composition of the present invention is suitable for the application against diseases or conditions in which a vascular endothelial cell-damaging factor, such as glycated protein (AGE), ox-LDL (also referred to in the present invention as oxidized or degenerated LDL), inflammatory cytokine (e.g., IL-1 or TNF-$\alpha$), and free radical, is activated. Glycated proteins (AGEs) are produced in the blood vessel of a patient with a high blood glucose level due to diabetes or the like, and damage vascular endothelial cells. Specific examples of the disease or condition in which a vascular endothelial cell-damaging factor is activated include a high glycated-protein (AGE) level, diabetes, hyperglycemia, a diabetic complication, arteriosclerosis obliterans (ASO) complicated with diabetes, a high ox-LDL level, hyperlipidemia involving a high ox-LDL level, abnormality of an inflammation marker (CRP, high-sensitivity CRP, interleukin 1, interleukin 6, TNF-$\alpha$, serum vascular cell adhesion molecule (VCAM), or the like), metabolic syndrome, systemic inflammatory response syndrome (SIRS), myocardial infarction, cerebral infarction, angina pectoris, and arteriosclerosis obliterans (ASO).

The inventive composition may be administered to a patient presenting any one or more out of a high glycated-protein (AGE) level, a high ox-LDL level, inflammation marker abnormality and hyperglycemia upon measurement of any one or more out of a glycated protein (AGE), ox-LDL, inflammation marker (CRP, high-sensitivity CRP, interleukin 1, interleukin 6, TNF-$\alpha$, serum vascular cell adhesion molecule (VCAM), or the like), and the blood glucose level. In that case, both the terms "high level" and "abnormality" indicate that a measured value exceeds the reference value specified with respect to the relevant check item, or, if the reference is not specified, that a measured value does not fall within a range of $\pm 15\%$ deviation from the mean value obtained among healthy people. In the present invention, the inflammatory marker is preferably CRP, high-sensitivity CRP, interleukin 1, interleukin 6, TNF-$\alpha$, or serum vascular cell adhesion molecule (VCAM).

The composition of the present invention is suitable for the application against diseases or conditions with damage to the vascular endothelium, and exemplary target diseases include, apart from those mentioned above, blood access formation (shunting) in a patient under hemodialysis, respiratory diseases such as idiopathic pulmonary fibrosis, bronchial asthma and acute respiratory distress syndrome, autoimmune diseases such as rheumatoid arthritis, polymyositis, systemic sclerosis, polyarteritis, systemic lupus erythematosus, autoimmune angiitis, Kawasaki disease, psoriasis, Wegener's granulomatosis, Behcet's disease, sarcoidosis and Graves' disease, heart diseases such as unstable angina pectoris, myocardial infarction, rheumatic heart disease, bypass operation and mitral valve replacement, lifestyle-related diseases such as arteriosclerosis, hyperlipidemia, diabetes and hypertriglyceridemia, malignant tumors such as melanoma, gastric cancer and laryngeal cancer, organ transplantation such as heart transplantation, liver transplantation, kidney transplantation and myeloid stem cell transplantation, as well as various infections, sepsis, DIC, and MOF. Included more preferably are diabetic complications and complications involved with organ transplantation, in particular myeloid stem cell transplantation, whereupon diabetic retinopathy, diabetic nephropathy and diabetic neuropathy are even more preferred examples.

The composition of the present invention may be applied to patients suffering from a disease or condition whose amelioration requires suppression of platelet aggregation in spite of a risk of bleeding. Examples of the disease whose amelioration requires suppression of platelet aggregation in spite of a risk of bleeding include such a disease as stroke involving lesions with bleeding due to the vascular rhexis caused by thrombi or emboli, and a disease subjected to the above-mentioned cardiac revascularization as a surgical procedure.

Patients who may bleed include, apart from those suffering from the above diseases, patients with hemophilia, with vitamin K deficiency, and a patient who is liable to bleed on an account of the vessel wall made fragile, or blood vessel made more breakable, or vascular permeability made higher, for some reason (patient with, for instance, vascular purpura, collagen disease, infection, allergic disease, inflammatory condition, or senile decay).

The composite composition of the present invention for preventing or treating a thrombus- or embolus-associated disease is effective for the prevention or treatment of cardiovascular death, fatal myocardial infarction, sudden cardiac death, non-fatal myocardial infarction, recurrence of myocardial infarction, angina pectoris decubitus, unstable angina pectoris, transient ischemic attack, congestive heart failure, new onset of exertional angina pectoris, destabilization of angina pectoris, ischemic heart disease, postoperative restenosis after cardiac revascularization, cardiovascular events occurring in an unstable phase after cardiac revascularization, cardiovascular events in a patient beyond an unstable phase after cardiac revascularization, as well as ulcers, pain and cryesthesia associated with arteriosclerosis obliterans.

The inventive composition is also effective for the treatment and/or prevention of stroke, namely a condition in which consciousness disorder or a nervous symptom occurs abruptly due to a cerebrovascular accident. The composition is particularly effective for the treatment and/or prevention of such conditions as recurrence of stroke in a patient with history of stroke, recurrence after an ischemic cerebrovascular accident, intracerebral hemorrhage (hypertensive intracerebral hemorrhage etc.), cerebral infarction, transient cerebral ischemic attack, subarachnoid hemorrhage, cerebral thrombosis (atherothrombotic cerebral infarction etc.), cerebral embolism (cardiogenic cerebral embolism etc.), lacunar infarction, and syncope.

The composition of the present invention for preventing or treating a thrombus- or embolus-associated disease is also effective for the suppression and treatment of a thrombotic event derived from the myocardial infarction which has developed recently, the suppression and treatment of a thrombotic event derived from the stroke or peripheral arterial disease which has developed recently, the suppression and treatment of a thrombotic event of acute coronary syndrome and so forth, the prevention and treatment of an atherothrombotic event in a patient suffering from myocardial infarction (within several to 35 days after onset), an ischemic cerebrovascular accident (within seven days to six months after onset), or a peripheral arterial disease, the prevention and treatment of an atherothrombotic event in a patient suffering from non-ST-elevation acute coronary syndrome (unstable angina pectoris or non-Q-wave myocardial infarction), or the like.

The composition of the present invention is effective for the prevention or treatment of such diseases as above, or the prevention of their recurrence, in mammals. The mammals are exemplified by humans, dogs, and cats, while the preferred are humans.

When the composition of the present invention is to be applied by combining two formulations prepared with two drugs, respectively, the drugs are each formulated in a known manner.

The composition of the present invention may contain a pharmaceutically acceptable vehicle in addition to active ingredients. Since EPA-E and DHA-E are of a highly unsaturated nature, it is desirable to allow the composition to contain an effective amount of an antioxidant, such as buthylated hydroxytoluene, buthylated hydroxyanisol, propyl gallate, gallic acid, a pharmaceutically acceptable quinone, and α-tocopherol. The inventive composition may also contain a known corrigent, flavoring agent, preservative, antioxidant, emulsifier, pH-adjusting agent, buffer, colorant or the like as appropriate. Specifically, the composition may contain as an excipient lactose, partly pregelatinized starch, hydroxypropylcellulose, macrogol, tocopherol, hydrogenated oil, sucrose ester of fatty acid, hydroxypropylmethylcellulose, titanium oxide, talc, dimthylpolysiloxane, silicon dioxide, carnauba wax, or the like.

The dosage form of a formulation is not particularly limited, and the inventive composition may be administered to a patient as an oral formulation in the form of tablet, film-coated tablet, capsule, microcapsule, granule, fine granule, powder, oral liquid preparation, syrup, jelly or the like, or as a parenteral formulation in the form of external preparation, such as injection, transfusion solution, and endermic preparation. For those patients who are able to take oral formulations, easy-to-take oral formulations are desirable, so that oral administration of a formulation as included in a capsule such as soft capsule and microcapsule, in tablet form, or in film-coated tablet form is particularly preferred.

The composition of the present invention can be prepared as a composite formulation containing, as active ingredients, at least one selected from the group consisting of clopidogrel and its pharmaceutically acceptable salts and at least one selected from the group consisting of icosapentaenoic acid as well as its pharmaceutically acceptable salts and esters. The composite formulation may contain an ω-3 long-chain unsaturated fatty acid other than EPA-E as an active ingredient, which ω-3 fatty acid is not particularly limited, but is desirably DHA-E. The composite formulation may also contain a third drug as an active ingredient. The third drug is not particularly limited, while it is preferable that the third drug does not reduce the effects of the present invention, and examples of such a drug include HMG-CoA reductase inhibitor, aspirin, and hypotensive agent.

The composite formulation is not particularly limited in dosage form, and it may be administered to a patient as an oral formulation in the form of tablet, film-coated tablet, capsule, microcapsule, granule, fine granule, powder, oral liquid preparation, syrup, jelly or the like, or as a parenteral formulation in the form of external preparation, such as injection, transfusion solution, and endermic preparation. In addition, the composite formulation includes a formulation made adapted for extended release, a formulation releasing two drugs separately with a certain time lag, and so forth.

The composite formulation of the present invention may contain a pharmaceutically acceptable vehicle in addition to active ingredients. The formulation may also contain a known antioxidant, coating agent, gelling agent, corrigent, flavoring agent, preservative, antioxidant, emulsifier, pH-adjusting agent, buffer, colorant or the like as appropriate.

The composite formulation of the present invention can be prepared according to a usual manner. Powder of ethyl icosapentate is obtained by a known method in which, for instance, an oil-in-water emulsion containing (A) ethyl icosapentate, (B) dietary fiber, (C) a starch hydrolysate and/or a reducing starch decomposition product obtained by saccharification into oligosaccharide, and (D) a water-soluble antioxidant is dried in a high vacuum, then pulverized (JP 10-99046 A). By using the powder of ethyl icosapentate thus obtained and clopidogrel sulfate powder, a formulation in the form of granule, fine granule, powder, tablet, film-coated tablet, chewable tablet, tablet for extended release, orally-disintegrating tablet (OD tablet) or the like can be prepared according to a usual manner. Chewable tablets may be obtained by the known method in which ethyl icosapentate is emulsified in a solution of water-soluble polymer such as hydroxypropylmethylcellulose, and the resultant emulsion is sprayed onto lactose or other excipient to form powdery glanules (JP 8-157362 A), with the granules being mixed with clopidogrel sulfate powder for compressing. Tablets for extended release may be obtained by (1) forming two layers containing ethyl icosapentate and clopidogrel sulfate, respectively, so as to arrange one layer inside and the other outside, or (2) forming two matrix disks containing the two ingredients, respectively, so as to layer them, or (3) embedding particulate capsules including one ingredient into the matrix containing the other ingredient, or (4) mixing the two drugs together, then subjecting the mixture to some measures for extended release. It is desirable that the active ingredients are each regulated in releasing rate, and the two drugs may be released simultaneously or separately with a certain time lag. Orally-disintegrating tablets may be produced in accordance with such a known method as disclosed in JP 8-333243 A, and a film preparation for oral cavity may be produced in accordance with such a known method as disclosed in JP 2005-21124 A. Since clopidogrel sulfate does not dissolve in EPA-E in a simple manner, the preparation of a formulation in the form of soft capsule, liquid preparation or the like requieres the measures as described in Examples. The composite formulation of the present invention thus encompasses formulations in which some measures are taken to compound clopidogrel sulfate and EPA-E into one formulation.

It is desirable that the active ingredients of the composite formulation of the present invention are released and absorbed so that their pharmacological actions may develop. Preferably, the composite formulation of the present invention has at least one effect out of an improved active-ingredient release, enhancement of the absorbability of active ingredients, enhancement of the dispersibility of active ingredients, an improved storage stability of the formulation in itself, and enhancement of the convenience to patients taking the formulation, or improvement of the compliance of such patients.

The dose and dosage period of EPA used in the composition of the present invention are made adequate to the expected actions of the drug, and modified appropriately depending on the dosage form, dosage route, frequency of administration per day, condition severity, body weight and age of a patient, and so forth. In the case of oral administration, a dose of 0.3 to 6 g/day, preferably 0.9 to 3.6 g/day, more preferably 1.8 to 2.7 g/day as EPA-E dose is divided into three parts to be administered separately, while the entire dose may be administered at a time or the dose may be divided into several parts, as required. Administration is preferably performed between meals or after meal, with the administration immediately after meal (within 30 minutes after meal) being more preferred. The period of oral administration at the above dose is allowed to last at least one year, preferably at least two years, more preferably at least 3.5 years, and even more preferably at least 5 years, whereupon administration should desirably be continued while a high risk of cardiovascular event occurrence and/or recurrence remains. If necessary, a drug withdrawal period of one day to about three months, preferably of one week to one month, may be provided.

In the composition of the present invention, clopidogrel sulfate is preferably used under the directions for use and dose of the drug alone, and its type, dosage form, dosage route, and frequency of administration per day are modified appropriately depending on the condition severity, body weight, sexuality and age of a patient, and so forth. In the case of oral administration, a dose of 0.05 to 300 mg/day, preferably 0.1 to 100 mg/day as clopidogrel dose is administered at a time or divided into two parts to be administered separately, while the dose may be divided into several parts as required. This compound may be administered orally at a higher dose (e.g., 100 to 300 mg) than the recommended daily dose (e.g., 75 mg) on the first day of administration, then at the recommended daily dose as a maintenance dose in accordance with the instructions of a doctor, if any. It is also possible to reduce the dose of clopidogrel sulfate in response to the dose of EPA-E.

In the present invention, at least one selected from the group consisting of clopidogrel and its pharmaceutically acceptable salts and at least one selected from the group consisting of icosapentaenoic acid as well as its pharmaceutically acceptable salts and esters may be applied in combination by any method as long as therapeutic effects are attained owing to the combined application of drugs. In a desirable application method, the therapeutic effect attained with clopidogrel sulfate and ethyl icosapentate applied in combination is more significant than the sum of the therapeutic effects attained with clopidogrel sulfate and with ethyl icosapentate, respectively, each drug being applied at the same dose as the dose upon application in combination. In this regard, therapeutic effects are not particularly limited as long as they are effects of preventing or treating a thrombus- or embolus-associated disease, and are exemplified by the platelet aggregation-suppressing effect. Therapeutic effects may be monitored on the basis of other parameters related to platelet aggregation.

The dose of clopidogrel sulfate and/or ethyl icosapentate may be reduced as compared with conventional ones. In that case, side effects of the drug or drugs are suppressed advantageously. For instance, each drug may be applied at a dose inadequate for any therapeutic effect to be attained with the relevant drug alone.

In another desirable application method, the therapeutic effect attained with clopidogrel sulfate and ethyl icosapentate, which are applied in combination with the dose of clopidogrel sulfate and/or ethyl icosapentate being inadequate for any therapeutic effect to be attained with the relevant drug alone, is more significant than the sum of the therapeutic effects attained with clopidogrel sulfate and with ethyl icosapentate, respectively, each drug being applied at the same dose as the dose upon application in combination.

If clopidogrel sulfate is applied at a dose inadequate for any therapeutic effect to be attained with this drug alone, such a dose is not specified because it varies with occasional condition or physique of a patient. As an example, the daily dose of clopidogrel is determined to be 75 mg, a recommended daily dose, or less, preferably 1 mg or more but less than 75 mg, more preferably 5 mg or more but not more than 50 mg, and even more preferably 10 mg or more but not more than 25 mg.

If ethyl icosapentate is applied at a dose inadequate for any therapeutic effect to be attained with this drug alone, such a dose is not specified because it varies with occasional condition or physique of a patient. As an example, the daily dose of EPA-E is determined to be 0.3 g or more but less than 6 g, preferably 0.3 g or more but not more than 3.6 g, more preferably 0.3 g or more but not more than 1.8 g, and even more preferably 0.3 g or more but not more than 0.9 g.

The ratio between the doses of clopidogrel sulfate and ethyl icosapentate is not particularly limited. Taking the dose ratio used in Examples and expected effects into account, it is desirable to administer to a human 2,700 mg of ethyl icosapentate with respect to 50 mg of clopidogrel sulfate. The dose ratio of clopidogrel sulfate to ethyl icosapentate is preferably 1:1 to 1:100, more preferably 1:10 to 1:80, even more preferably 1:30 to 1: 60, especially 1:50 to 1:60, and most preferably 1:54. Also in the case where a composite formulation is prepared, the two drugs are preferably compounded at such a ratio.

The dose of at least one selected from the group consisting of clopidogrel and its pharmaceutically acceptable salts, and the dose of at least one selected from the group consisting of icosapentaenoic acid as well as its pharmaceutically acceptable salts and esters are modified appropriately through the inspection of platelet aggregation and/or bleeding time or the like. For instance, platelet aggregation is measured during the administration of clopidogrel sulfate alone, then, using the measurements as indices, the dose of clopidotrel sulfate is reduced and administration of EPA-E is begun so as to attain the therapeutic effects of the present invention.

The bleeding time upon application of the inventive composition is preferably not longer than that found when clopidogrel sulfate is applied alone at the dose which is required in order to attain the same therapeutic effects as the present invention.

Depending on the condition of a patient, the composition of the present invention may be applied appropriately in combination with at least one selected from among other drugs including antiplatelet agents such as aspirin, ticlopidine, cilostazol and prasugrel; anticoagulants such as warfarin, heparin, ximelagatran and enoxaparin sodium; therapeutic agents for hypertension, such as angiotensin II receptor antagonist (candesartan, losartan, valsartan, irbesartan, etc.), angiotensin converting enzyme inhibitor, calcium channel antagonist (amlodipine, cilnidipine, etc.) and $\alpha_1$-blocker; antidiabetic agents, or drugs for ameliorating glucose tolerance abnormality, such as $\alpha$-glucosidase inhibitor (voglibose, acrabose, etc.), biguanide-type drug, thiazolidinedione-type drug (pioglitazone, rosiglitazone, rivoglitazone, etc.), rapid-acting insulin secretagogue (mitiglinide, nateglinide, etc.) and insulin; antilipemics such as HMG-CoA RI as referred to above (atorvastatin, simvastatin, pravastatin, rosuvastatin calcium, etc.), fibrate-type drug, squalene synthase inhibitor (TAK-475 etc.) and cholesterol absorption inhibitor (ezetimibe etc.); and COX-2 inhibitors (celecoxib etc.). To be more convenient for use, the inventive composition may also be packaged together with a HMG-CoA RI and/or at least one other drug as above. The inventive composition is particularly applicable in combination with aspirin.

In its another aspect, the present invention provides a platelet aggregation inhibitor which includes, as active ingredients to be applied in combination, at least one selected from the group consisting of clopidogrel and its pharmaceutically acceptable salts and at least one selected from the group consisting of icosapentaenoic acid as well as its pharmaceutically acceptable salts and esters. Preferably, the formulation is for the suppression of platelet aggregation with collagen, and includes, as active ingredients to be applied in combination, clopidogrel sulfate and ethyl icosapentate. This formulation is identical to the composition for prevention or treatment as described above in active ingredient, preparation process, and usage.

While description is made chiefly on clopidogrel sulfate and ethyl icosapentate in the above, other salts and esters which are pharmaceutically acceptable are also usable.

EXAMPLES

The effects of the composition of the present invention will be illustrated by the experiment and examples as below.

1. Test drugs

EPA-E of 98.4% purity purchased from Nippon Suisan Kaisha, Ltd. was emulsified in a 5% aqueous solution of gum arabic using a disperser and an ultrasonic homogenizer to provide the drug's solution for administration. Clopidogrel (Plavix™ tablets from sanofi-aventis K.K.) was suspended in a 5% aqueous solution of gum arabic to provide the drug's solution for administration.

2. Experimental animals

Male Japanese white rabbits (Kbs:JW) (weighing 2.50 to 2.99 kg each) were purchased from KITAYAMA LABES Co., Ltd. They were kept separately in animal cages at a temperature of 23 $\pm$ 2°C, a relative humidity of 55 $\pm$ 15%, and with a light-dark cycle of 12 hours. After a preliminary keeping for at least 12 days, the rabbits were subjected to trial. The animals were permitted to take RC-4 feed (Oriental Yeast Co., Ltd.) and tap water freely. Animal experiments had been approved in advance by the ethics committee on animal experiments set up in the General Research Institute of Mochida Pharmaceutical Co., Ltd. and were conducted in accordance with the "Animal Experimentation Rules in Consideration of Animal Welfare" established by the committee.

3. Drug administration and analysis of platelet aggregation

After the preliminary keeping, the rabbits were randomly divided into four groups, namely, control group, EPA-E group, clopidogrel group, and combination group. To the EPA-E group and the combination group, EPA-E was orally administered once a day for four weeks at a dose of 300 mg/kg. To the control group and the clopidogrel group, a 5% aqueous solution of gum arabic was orally administered instead of EPA-E. After the last EPA-E or gum arabic administration, the rabbits were fasted overnight, then clopidogrel was orally administered to the clopidogrel group and the combination group at a single dose of 20 mg/kg. To the control group and the EPA-E group, the 5% aqueous solution of gum arabic was orally administered instead of clopidogrel. Two hours after the clopidogrel administration, blood was sampled using a syringe containing citric acid in an amount of a tenth of the syringe volume, and the white blood cell count, red blood cell count, hemoglobin, hematocrit, and platelet count were measured on an automated hematology analyzer (type F-820 from Sysmex Corporation). The blood was centrifuged (at 150 xg and at room temperature for 15 minutes) immediately after the above measurement to obtain platelet-rich plasma (PRP). The PRP was further centrifuged (at 1,500 xg and at room temperature for 15 minutes) to collect platelet-poor plasma (PPP), and the platelet count was regulated with the PRP and PPP so that it might be $3.2 \times 10^5$/mL (final level). The PRP was kept at 37°C for four minutes, then an ADP (adenosine 5'-diphosphate) solution with a final concentration of 3 $\mu$M (MC Medical, Inc.) or a collagen solution with a final concentration of 6 $\mu$g/mL (Nycomed) was added thereto as an aggregation inducer so as to observe platelet aggregation using a platelet aggregation analyzer (MCM HEMA TRACER 313M from MC Medical, Inc.). The platelet aggregation activity was evaluated on the basis of maximum aggregation rate, and the suppressibility of platelet aggregation was calculated according to the following equation.

$$\text{Aggregation suppressibility (\%)} =$$
$$\{[\text{maximum aggregation rate (mean) in control group}] -$$
$$[\text{maximum aggregation rate (mean) in experimental group}]\} /$$
$$[\text{maximum aggregation rate (mean) in control group}] \times 100.$$

4. Measurement of coagulation-related parameters

Platelet-poor plasma (PPP) was measured in terms of prothrombin time (PT) and activated partial thromboplastin time (APTT) as coagulation-related parameters. Simplastin Excel (bioMerieux Japan, Ltd.) was used for the measurement of PT, and Data-Fi APTT (Sysmex Corporation) for the measurement of APTT. The parameters were measured on a fully-automated blood coagulation analyzer (AMAX CS-190. from MC Medical, Inc.).

5. Measurement of bleeding time

The bleeding time was measured according to the method of Elg et al. (Thromb. Res. 2001; 101(3): 159-170). To be more specific: the right ear of a rabbit under pentobarbital anesthetic (Somnopentyl™ from Kyoritsu Seiyaku Corporation) was punctured with a scalpel to give therein a certain wound. The blood shed from the puncture wound was blotted every 15 seconds with a filter paper, and the bleeding time was determined as the time which lapsed away before no blood was adhered to a filter paper any more. Every rabbit had two puncture wounds, and the bleeding time was obtained for each rabbit as the mean of the bleeding times measured on the wounds, respectively. The bleeding time measurement was performed in a blind manner.

6. Statistical analysis

The measurement results set forth for each group are each the mean of the values obtained in 8 or 9 cases plus/minus a standard error. The results were compared between the groups using the Tukey-Kramer method for multiple comparison testing, and the statistical significance was confirmed below a set level of 5%.

<Results>

1. Effects on ADP-induced platelet aggregation

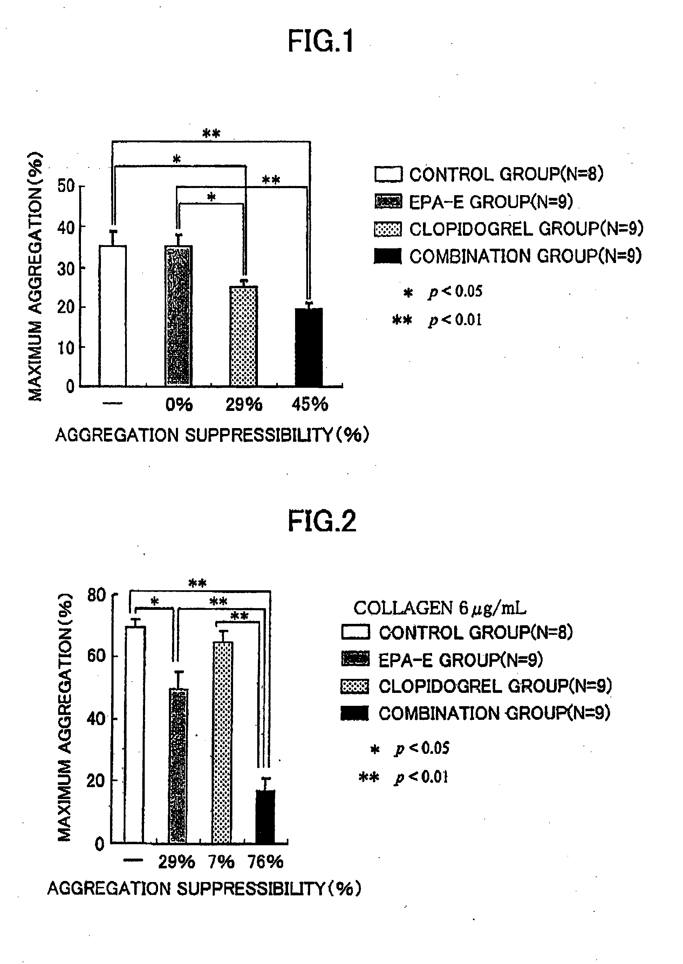

FIG. 1 shows effects on the platelet aggregation induced by ADP. Administration of EPA-E alone had no influence on the maximum aggregation rate with an aggregation suppressibility of 0%. Administration of clopidogrel alone reduced the maximum aggregation rate with an aggregation suppressibility of 29%, so that a statistically significant difference was seen between the clopidogrel group and the control group. In the group to which EPA-E and clopidogrel were administered in combination, the aggregation suppressibility was 45%. From the results as above, application of the two drugs in combination proved effective.

2. Effects on collagen-induced platelet aggregation

FIG. 2 shows effects on the platelet aggregation induced by collagen. Administration of EPA-E alone reduced the maximum aggregation rate significantly in a statistical sense, whereupon the aggregation suppressibility was 29%. When clopidogrel was administered alone, no differences were seen which were statistically significant. The aggregation suppressibility was 7%. On the other hand, combined administration of EPA-E and clopidogrel greatly reduced the maximum aggregation rate, that is to say, the maximum aggregation rate found in the combination group with respect to the aggregation which had been induced by 6 $\mu$g/mL of collagen was significantly low in a statistical sense as compared with either of those in the groups to which the two drugs were administered by themselves, respectively. The aggregation suppressibility in the combination group was 76%. The results as above proved that synergism is achieved on the collagen-induced platelet aggregation by applying EPA-E and clopidogrel in combination, and the application of the two drugs in combination potentiates the platelet aggregation-suppressing action. The effect of suppressing platelet aggregation will be expected even from the clopidogrel administered at such a low dose as generally producing no effect, if the drug is applied in combination with EPA-E.

3. Effects on coagulation-related parameters

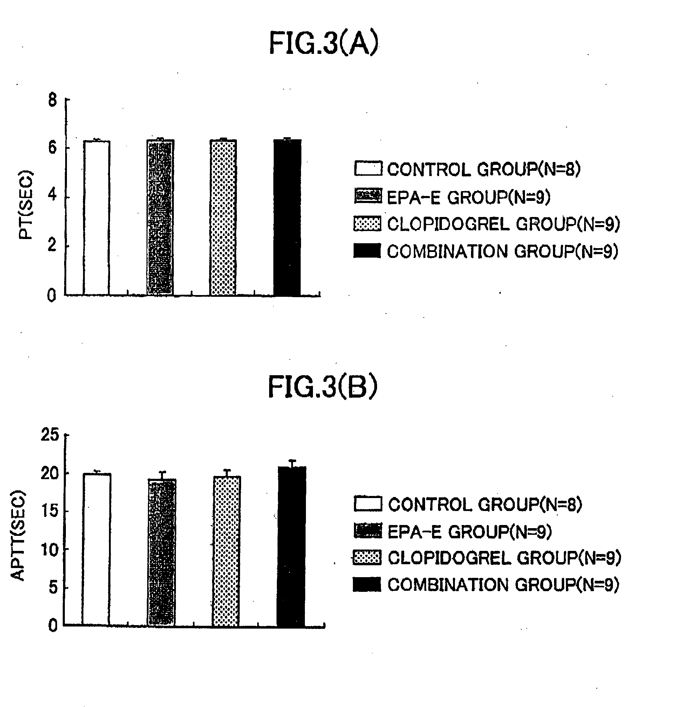

FIGs. 3(A) and 3(B) show effects on coagulation-related parameters. In any of the groups to which EPA-E and clopidogrel were administered by themselves, respectively, and the group to which the two drugs were administered in combination, neither PT (prothrombin time) nor APTT (activated partial thromboplastin time) had a statistically significant change as compared with the control group.

4. Effects on bleeding time

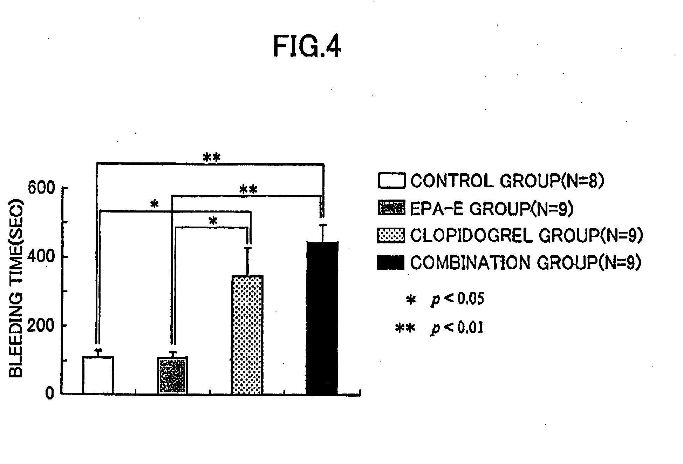

FIG. 4 shows effects on the bleeding time. While administration of EPA-E alone had no influence on the bleeding time, administration of clopidogrel alone prolonged the bleeding time significantly in a statistical sense. When the two drugs were administered in combination, the bleeding time was not prolonged significantly in a statistical sense as compared with that found when clopidogrel was administered alone. The results as above proved that the application of EPA-E and clopidogrel in combination has little side effect of prolonging the bleeding time, and the two drugs can safely be combined with each other.

5. Effects on hematological parameters

Table 1 shows effects on hematological parameters. In any of the groups to which EPA-E and clopidogrel were administered by themselves, respectively, and the group to which the two drugs were administered in combination, any of the white blood cell count, red blood cell count, hemoglobin, hematocrit and platelet count had not a statistically significant change as compared with the control group.

As a result of the above experiment, it was found that the combined application of EPA-E and clopidogrel sulfate potently suppresses platelet aggregation and, at the same time, does not cause a significant change in bleeding time as compared with EPA-E alone or clopidogrel alone. Consequently, EPA-E and clopidogrel sulfate will also be more potent against thrombogensesis if applied in combination, and their application in combination is useful for the prevention or treatment of a wide variety of thrombus- or embolus-associated diseases. Since the combined application of EPA-E and clopidogrel sulfate had a suppressive synergism on the platelet aggregation induced by collagen adhesion, it is suggested in particular that the combined application as above is effective against diseases or conditions involving the platelet activation or aggregation which is induced by the collagen exposed due to vascular endothelial cell damage. The combined application of the two drugs is considered to be also effective against a disease whose amelioration requires suppression of platelet aggregation in spite of a higher risk of bleeding than that under healthy conditions, for instance, a disease or condition with vascular endothelial cell damage due to a mechanical factor such as revascularization and surgical procedures. The combined application of EPA-E and clopidogrel sulfate is suitably employed if the dose of clopidogrel sulfate is to be set lower than usual (in order to reduce side effects of clopidogrel, for instance).

[Table 1]

| Effects of EPA-E and Clopidogrel on Hematological Parameters | | | | |
|---|---|---|---|---|
| | Control group (N=8) | EPA-E group (N=9) | Clopidogrel group (N=9) | Combination group (N=9) |
| White blood cell count ($\times 10^2/\mu$L) | 53.8 $\pm$ 2.1 | 58.8 $\pm$ 5.2 | 58.0 $\pm$ 4.8 | 60.9 $\pm$ 5.9 |
| Red blood cell count ($\times 10^4/\mu$L) | 570 $\pm$ 29 | 563 $\pm$ 10 | 584 $\pm$ 17 | 574 $\pm$ 15 |
| Hemoglobin (g/dL) | 13.3 $\pm$ 0.5 | 13.1 $\pm$ 0.2 | 13.8 $\pm$ 0.4 | 13.3 $\pm$ 0.3 |
| Hematocrit (%) | 38.0 $\pm$ 1.6 | 37.3 $\pm$ 0.6 | 39.3 $\pm$ 1.2 | 38.4 $\pm$ 0.9 |
| Platelet count ($\times 10^4/\mu$L) | 36.2 $\pm$ 2.5 | 30.4 $\pm$ 1.2 | 38.0 $\pm$ 2.3 | 33.8 $\pm$ 1.6 |
| Significant difference is seen between the groups (p<0.05). | | | | |

Composite formulations containing clopidogrel sulfate and ethyl icosapentate are prepared according to a usual manner.

[Table 2]

| <Formulation Example 1 (soft capsules)> | |
|---|---|
| Component | Amount per capsule |
| Ethyl icosapentate | 300 mg |
| Clopidogrel sulfate | 16.3 mg |
| Gelatin | 170 mg |
| D-Sorbitol | 25 mg |
| Concentrated glycerin | 25 mg |
| Sodium hydroxide | q.s. |
| Purified water | q.s. |

According to the composition as set forth in Table 2, concentrated glycerin, clopidogrel sulfate and purified water are mixed together and agitated, with the pH being adjusted to about 7 with sodium hydroxide. Gelatin and D-sorbitol are added to the liquid mixture and dissolved therein by the heating to 60°C under agitation. The resultant solution is degassed under a reduced pressure, and regulated in viscosity with purified water to obtain a solution for soft capsule shell formation. By using the solution for soft capsule shell formation and ethyl icosapentate, soft capsules each containing 300 mg of ethyl icosapentate and 12.5 mg of clopidogrel are obtained.

Following a similar procedure, soft capsules each containing 300 mg of ethyl icosapentate and 5.6 mg of clopidogrel are obtained by using 300 mg of ethyl icosapentate and 7.2 mg of clopidogrel sulfate per capsule.

[Table 3]

| <Formulation Example 2 (soft capsules)> | | |
|---|---|---|
| Component | | Amount per capsule |
| A | Ethyl icosapentate | 300 mg |
| | Clopidogrel sulfate | 16.3 mg |
| B | Gelatin | 170 mg |
| | D-Sorbitol | 25 mg |
| | Concentrated glycerin | 25 mg |
| | Purified water | s.q. |

According to the composition B as set forth in Table 3, water is added to concentrated glycerin, to which gelatin and D-sorbitol are added and dissolved therein by the heating to 60°C under agitation. The resultant solution is degassed under a reduced pressure, and regulated in viscosity with purified water to obtain a solution for soft capsule shell formation. Next, according to the composition A, pulverized clopidogrel sulfate is mixed into ethyl icosapentate to obtain a uniform dispersion as a liquid content for soft capsules. By using the solution for soft capsule shell formation and liquid content for soft capsules thus obtained, soft capsules each containing 300 mg of ethyl icosapentate and 12.5 mg of clopidogrel are obtained.

[Table 4]

| <Formulation Example 3 (liquid)> | | |
|---|---|---|
| Component | | Amount per pack |
| A | Ethyl icosapentate | 1800 mg |
| | Orange oil | 81 mg |
| B | Clopidogrel sulfate | 97.9 mg |
| | Polyoxyethylene (105)-polyoxypropylene (5) glycol | 144 mg |
| | Trehalose | 1350 mg |
| | Ascorbyl stearate | 1.8 mg |
| | Sodium erythorbate | 117 mg |
| C | Sodium hydroxide | s.q. |
| | Purified water | s.q. |
| Sum | | 9 g |

According to the composition B as set forth in Table 4, the listed components are added to purified water and dissolved therein, with the pH being adjusted to about 7 with sodium hydroxide. To the resultant solution, the components listed in the composition A are added, and the emulsion obtained by a rapid agitation of the solution under a reduced pressure is poured into a plurality of stick packages made of an aluminum-laminated film in an amount of 9 g each. The inside of the packages is subjected to nitrogen replacement before the packages are sealed. The liquid formulation which contains 1800 mg of ethyl icosapentate and 75 mg of clopidogrel per pack is thus obtained.

[Table 5]

| <Formulation Example 4 (jelly)> | | |
|---|---|---|
| Component | | Amount per pack |
| A | Ethyl icosapentate | 1800 mg |
| | Orange oil | 81 mg |

(continued)

| <Formulation Example 4 (jelly)> | | |
|---|---|---|
| Component | | Amount per pack |
| B | Clopidogrel sulfate | 97.9 mg |
| | Polyoxyethylene (105)-polyoxypropylene (5) glycol | 144 mg |
| | Trehalose | 1350 mg |
| | Ascorbyl stearate | 1.8 mg |
| | Sodium erythorbate | 117 mg |
| | Pullulan | 270 mg |
| C | Carrageenan | 37.8 mg |
| | Carob bean gum | 22.5 mg |
| | Concentrated glycerin | 675 mg |
| D | Sodium hydroxide | s.q. |
| | Purified water | s.q. |
| Sum | | 9 g |

According to the composition B as set forth in Table 5, the listed components are added to purified water and dissolved therein, with the pH being adjusted to about 7 with sodium hydroxide. To the resultant solution, the components listed in the composition A are added, and an emulsion is obtained by a rapid agitation of the solution under a reduced pressure. The emulsion is heated to 85°C, then the uniform dispersion which is provided by mixing the components in the composition C under agitation is added to the emulsion, and the mixture is agitated to uniformity. The liquid preparation thus obtained is poured into a plurality of stick packages made of an aluminum-laminated film in an amount of 9 g each. The inside of the packages is subjected to nitrogen replacement before the packages are sealed. After cooling to solidification, the jelly formulation which contains 1800 mg of ethyl icosapentate and 75 mg of clopidogrel per pack is obtained.

[Table 6]

| <Formulation Example 5 (composite formulation for divided packaging)> | |
|---|---|
| Component | Amount per pack |
| Clopidogrel sulfate | 97.9 mg |
| Lactose | 147.1 mg |
| Cornstarch | 25 mg |
| Crystalline Cellulose | 80 mg |
| Magnesium stearate | 10 mg |

According to the composition as set forth in Table 6, 24 mg tablets (each containing 5 mg of clopidogrel) are produced. Apart from the tablets, seamless soft capsules of about 4 mm in diameter with gelatin shell are produced each of which contains 20 mg of ethyl icosapentate. The clopidogrel-containing tablets and the ethyl icosapentate-containing seamless soft capsules are placed in a plurality of stick packages made of an aluminum-laminated film, 15 tablets and 90 capsules each in number. The inside of the packages is subjected to nitrogen replacement before the packages are sealed. The composite formulation which is dividedly packaged and contains 1800 mg of ethyl icosapentate and 75 mg of clopidogrel per pack is thus obtained.

By placing 10 clopidogrel-containing tablets and 135 ethyl icosapentate-containing seamless soft capsules in a stick package made of an aluminum-laminated film, and subjecting the inside of the package to nitrogen replacement before the package is sealed, the composite formulation which is dividedly packaged and contains 2700 mg of ethyl icosapentate and 50 mg of clopidogrel per pack is obtained.

**Claims**

1. A composition for use in the prevention or treatment of a thrombus- or embolus-associated disease comprising, as active ingredients to be applied in combination, at least one selected from the group consisting of clopidogrel and its pharmaceutically acceptable salts and at least one selected from the group consisting of icosapentaenoic acid as well as its pharmaceutically acceptable salts and esters, wherein the application in combination includes administering a formulation containing both active ingredients, and administering the two active ingredients as separate formulations simultaneously, or separately with a certain time lag.

2. The composition for use in the prevention or treatment according to claim 1, wherein said composition is administered to a subject suffering from a disease or condition with vascular endothelial cell damage.

3. The composition for use in the prevention or treatment according to claim 2, wherein said disease or condition with vascular endothelial cell damage is a disease or condition which includes at least one symptom selected from the group consisting of high glycated-protein (or AGE) levels, diabetes, hyperglycemia, diabetic complications, arteriosclerosis obliterans (ASO) complicated with diabetes, high ox-LDL levels, hyperlipidemia involving high ox-LDL Levels, and inflammation marker abnormality.

4. The composition for use in the prevention or treatment according to any one of claims 1 through 3, wherein said composition is administered to a subject suffering from a disease or condition whose amelioration requires suppression of platelet aggregation in spite of a risk of bleeding.

5. The composition for use in the prevention or treatment according to any one of claims 1 through 4, wherein said composition is administered to a subject selected from the group consisting of a subject having undergone revascularization and a subject with history of stroke.

6. The composition for use in the prevention or treatment according to any one of claims 1 through 5, wherein said thrombus- or embolus-associated disease comprises at least one selected from the group consisting of thrombosis, embolism, cardiovascular death, fatal myocardial infarction, sudden cardiac death, non-fatal myocardial infraction, recurrence of myocardial infraction, angina pectoris decubitus, unstable angina pectoris, transient ischemic attack, congestive heart failure, new onset of exertional angina pectoris, destabilization of angina pectoris, ischemic heart disease, postoperative restenosis after cardiac revascularization, cardiovascular events occurring in an unstable phase after cardiac revascularization, cardiovascular events in a patient beyond an unstable phase after cardiac revascularization, progression of atherosclerosis, stroke, recurrence of stroke in a patient with history of stroke, recurrence after an ischemic cerebrovascular accident, consciousness disorder or nervous symptoms caused by a cerebrovascular accident, cerebral infraction, transient cerebral ischemic attack, subarachnoid hemorrhage, cerebral thrombosis, cerebral embolism, lacunar infarct, syncope, ulcers, pain and cryesthesia associated with arteriosclerosis obliternas, or thrombotic and/or atherothrombotic events occurring in myocardial infraction, stroke, ischemic cerebrovascular accidents, peripheral arterial diseases, acute coronary syndrome and non-ST-elevation acute coronary syndrome (unstable angina pectoris or non-Q-wave myocardial infraction).

7. The composition for use in the prevention or treatment according to any one of claims 1 through 6, comprising clopidogrel sulfate and ethyl icosapentate as active ingredients.

8. The composition for use in the prevention or treatment according to any one of claims 1 through 7, wherein a dose ratio of clopidogrel sulfate to ethyl icosapentate is 1:10 to 1:80.

9. The composition for use in the prevention or treatment according to any one of claims 1 through 8, which is a composite formulation of clopidogrel sulfate and ethyl icosapentate.

10. The composition for use in the prevention or treatment according to any one of claims 1 through 9, wherein said composition is applied as a platelet aggregation inhibitor.

11. Use of at least one selected from the group consisting of clopidogrel and its pharmaceutically acceptable salts and at least one selected from the group consisting of icosapentaenoic acid as well as its pharmaceutically acceptable salts and esters for manufacturing a composition for prevention or treatment of a thrombus- or embolus-associated disease or condition in a subject suffering from a disease or condition with vascular endothelial cell damage to be applied in combination, wherein the application in combination includes administering a formulation containing both

**EP 2 119 443 B1**

active ingredients, and administering the two active ingredients as separate formulations simultaneously, or separately with a certain time lag.

12. At least one selected from the group consisting of icosapentaenoic acid as well as its pharmaceutically acceptable salts and esters for use in the prevention or treatment of a thrombus- or embolus-associated disease to be applied in combination with at least one selected from the group consisting of clopidogrel and its pharmaceutically acceptable salts simultaneously, or separately with a certain time lag.

13. At least one selected from the group consisting of clopidrogel and its pharmaceutically acceptable salts for use in the prevention or treatment of a thrombus- or embolus-associated disease to be applied in combination with at least one selected from the group consisting of icosapentaenoic acid as well as its pharmaceutically acceptable salts and esters simultaneously, or separately with a certain time lag.

**Patentansprüche**

1. Zusammensetzung zur Verwendung in der Vorbeugung oder Behandlung einer mit einem Thrombus oder einem Embolus verknüpften Krankheit, umfassend als wirksame Bestandteile, die in Kombination angewendet werden, mindestens eines ausgewählt aus der Gruppe bestehend aus Clopidogrel und seinen pharmazeutisch verträglichen Salzen und mindestens eines ausgewählt aus der Gruppe bestehend aus Eicosapentaensäure sowie ihrer pharmazeutisch verträglichen Salze und Ester, wobei die Anwendung in Kombination das Verabreichen einer Formulierung enthaltend beide wirksamen Bestandteile und das Verabreichen der zwei wirksamen Bestandteile gleichzeitig oder getrennt mit einem bestimmten zeitlichen Abstand als separate Formulierungen umfasst.

2. Die Zusammensetzung zur Verwendung in der Vorbeugung oder Behandlung gemäß Anspruch 1, wobei die Zusammensetzung einem Subjekt verabreicht wird, welches an einer Krankheit oder einem Zustand mit einem vaskulären Endothelzellschaden leidet.

3. Die Zusammensetzung zur Verwendung in der Vorbeugung oder Behandlung gemäß Anspruch 2, wobei die Krankheit oder der Zustand mit vaskulärem Endothelzellschaden eine Krankheit oder Zustand ist, welche(r) zumindest ein Symptom ausgewählt aus der Gruppe bestehend aus hohen Spiegeln an glykardierten Proteinen (oder AGE), Diabetes, Hyperglykämie, diabetischen Komplikationen, Arteriosklerose obliterans (ASO) erschwert mit Diabetes, hohen Spiegeln an oxLDL, Hyperlipidämie unter Einbezug von hohen Spiegeln an oxLDL und einer Anomalität der Entzündungsmarker beinhaltet.

4. Die Zusammensetzung zur Verwendung in der Vorbeugung oder Behandlung gemäß einem der Ansprüche 1 bis 3, wobei die Zusammensetzung einem Subjekt verabreicht wird, welches an einer Krankheit oder Zustand leidet, dessen Verbesserung die Unterdrückung der Blutplättchenaggregation trotz des Risikos einer Blutung erfordert.

5. Die Zusammensetzung zur Verwendung in der Vorbeugung oder Behandlung gemäß einem der Ansprüche 1 bis 4, wobei die Zusammensetzung einem Subjekt verabreicht wird, welches ausgewählt ist aus der Gruppe bestehend aus einem Subjekt, welches sich einer Revaskularisation unterworfen hat, und einem Subjekt mit Schlaganfallvergangenheit.

6. Die Zusammensetzung zur Verwendung in der Vorbeugung oder Behandlung gemäß einem der Ansprüche 1 bis 5, wobei die Thrombus- oder Embolus-verknüpfte Krankheit mindestens eine ausgewählt aus der Gruppe bestehend aus Thrombose, Embolie, Herz-Kreislauf-Tod, tödlicher Myokardinfarkt, plötzlicher Herztod, nicht-tödlicher Myokardinfarkt, Angina Pectoris Decubitus, instabile Angina Pectoris, transitorische ischämische Attacke, chronische Herzinsuffizienz, neuer Ausbruch einer Belastungsangina Pectoris, Destabilisierung einer Angina Pectoris, ischämische Herzkrankheit, nachoperative Restenose nach Herzrevaskularisation, Herz-Kreislauf-Vorfälle in einem Patienten, der über eine instabile Phase nach Herzrevaskularisation hinaus ist, Fortschritt von Atherosklerose, Schlaganfall, Wiederauftreten von Schlaganfall in einem Patienten mit Schlaganfallvergangenheit, Wiederauftreten nach einem Gehirnschlag, Bewusstseinsstörung oder nervöse Symptome verursacht durch einen Gehirnschlag, Gehirninfarkt, zerebrale transitorische ischämische Attacke, Subarachnoidalblutung, Hirnthrombose, Hirnembolie, lakunärer Hirninfarkt, Synkope, Ulcera, Schmerz- und Kälte-Überempfindlichkeit verbunden mit Arteriosklerose obliterans, oder thrombotische und/oder atherothrombotische Vorfälle, die beim Herzinfarkt, Schlaganfall, ischämische Gehirnschläge, periphere Arterienkrankheiten, akutem Koronarsyndrom und nicht-ST-Hebungskoronarsyndrom (akut) (instabile Angina Pectoris oder non-Q-wave Myokardinfarkt) auftreten, umfasst.

18

7. Die Zusammensetzung zur Verwendung in der Vorbeugung oder Behandlung gemäß einem der Ansprüche 1 bis 6, umfassend Clopidogrel-Sulfat und Ethyleicosapentat als wirksame Bestandteile.

8. Die Zusammensetzung zur Verwendung in der Vorbeugung oder Behandlung gemäß einem der Ansprüche 1 bis 7, wobei ein Dosierungsverhältnis von Clopidogrel-Sulfat zu Ethyleicosapentat 1:10 bis 1:80 beträgt.

9. Die Zusammensetzung zur Verwendung in der Vorbeugung oder Behandlung gemäß einem der Ansprüche 1 bis 8, welche eine Kompositformulierung aus Clopidogrel-Sulfat und Ethyleicosapentat ist.

10. Die Zusammensetzung zur Verwendung in der Vorbeugung oder Behandlung gemäß einem der Ansprüche 1 bis 9, wobei die Zusammensetzung als ein Blutplättchenaggregationshemmer verabreicht wird.

11. Verwendung von mindestens einem ausgewählt aus der Gruppe bestehend aus Clopidogrel und seinen pharmazeutisch verträglichen Salzen und mindestens einem ausgewählt aus der Gruppe bestehend aus Eicosapentaensäure sowie ihren pharmazeutisch verträglichen Salzen und Estern zur Herstellung einer Zusammensetzung für die Vorbeugung oder Behandlung einer Thrombus- oder Embolus-verknüpften Krankheit oder Zustand in einem Subjekt, welches an einer Krankheit oder einem Zustand mit vaskulärem Endothelzellschaden leidet, um in Kombination angewandt zu werden, wobei die Anwendung in Kombination das Verabreichen einer Formulierung enthaltend beide aktiven Bestandteile und das Verabreichen der zwei wirksamen Bestandteile gleichzeitig oder getrennt mit einem bestimmten zeitlichen Abstand als separate Formulierungen umfasst.

12. Mindestens eines ausgewählt aus der Gruppe bestehend aus Eicosapentaensäure sowie ihrer pharmazeutisch verträglichen Salze und Ester zur Verwendung in der Vorbeugung oder Behandlung einer Thrombus- oder Embolus-verknüpften Krankheit, um in Kombination mit mindestens einem ausgewählt aus der Gruppe bestehend aus Clopidogrel und seinen pharmazeutisch verträglichen Salzen gleichzeitig oder getrennt mit einem bestimmten zeitlichen Abstand angewandt zu werden.

13. Mindestens eines ausgewählt aus der Gruppe bestehend aus Clopidogrel und seinen pharmazeutisch verträglichen Salzen zur Verwendung in der Vorbeugung oder Behandlung einer Thrombus- oder Embolus-verknüpften Krankheit, um in Kombination mit mindestens einem ausgewählt aus der Gruppe bestehend aus Eicosapentaensäure sowie ihrer pharmazeutisch verträglichen Salze und Ester gleichzeitig oder getrennt mit einem bestimmten zeitlichen Abstand angewandt zu werden.

**Revendications**

1. Une composition pour utiliser dans la prévention ou le traitement de la maladie associé à un thrombus ou à un embolus comprenant comme ingrédients actifs à appliquer conjointement, au moins l'un sélectionné parmi le groupe consistant en du clopidogrel et ces sels pharmaceutiquement acceptable et au moins l'un sélectionné parmi le groupe consistant en l'acide icosapentaenoïque aussi bien que ces sels et esters pharmaceutiquement acceptables, dans laquelle l'application conjointe inclut l'administration d'une formulation contenant les deux ingrédients actifs et l'administration simultanée ou séparée par un certain laps de temps des deux ingrédients actifs comme formulations distinctes.

2. La composition pour utiliser dans la prévention ou le traitement selon la revendication 1, dans laquelle ladite composition est administrée à un sujet souffrant d'une maladie ou d'un état avec des dommages cellulaires de l'endothélium vasculaire.

3. La composition pour utiliser dans la prévention ou le traitement selon la revendication 2, dans laquelle ladite maladie ou ledit état avec des dommages cellulaires de l'endothélium vasculaire est une maladie ou un état qui inclut au moins un symptôme sélectionné parmi le groupe consistant en un niveau élevé de protéine glyquée (ou AGE), des diabètes, de l'hyperglycémie, des complications diabétiques, d'artérioscléroses oblitérantes (ASO) complexe avec le diabète, des niveaux élevés de ox-LDL, hyperlipidémie entrainant des niveaux élevés de ox-LDL, et anomalie des marqueurs d'inflammation.

4. La composition pour utiliser dans la prévention ou le traitement selon une quelconque des revendications 1 à 3, dans laquelle ladite composition est administrée à un sujet souffrant d'une maladie ou d'un état dont l'amélioration requiert la suppression d'agrégation plaquettaire en dépit d'un risque de saignement.

5. La composition pour utiliser dans la prévention ou le traitement selon une quelconque des revendications 1 à 4, dans laquelle ladite composition est administrée à un sujet sélectionné parmi le groupe consistant en un sujet ayant subi une revascularisation et un sujet avec des antécédents d'AVC.

6. La composition pour utiliser dans la prévention ou le traitement selon une quelconque des revendications 1 à 5, dans laquelle ladite maladie associé à un thrombus ou à un embolus comprend au moins une sélectionnée parmi le groupe consistant en la thrombose, l'embolie, la mort d'origine cardiovasculaire, l'infarctus du myocarde fatal, la mort subite d'origine cardiaque, l'infarctus du myocarde non fatal, la récurrence d'infarctus du myocarde, l'angine de poitrine decubitus, l'angine de poitrine dite « instable », l'accident ischémique transitoire, l'insuffisance cardiaque congestive, la nouvelle apparition à l'effort d'une angine de poitrine, la déstabilisation de l'angine de poitrine, la cardiopathie ischémique, la resténose post-opératoire après une revascularisation cardiaque, les événements cardiovasculaires survenant dans une phase instable après une revascularisation cardiaque, les événements cardiovasculaires chez un patient au-delà de la phase instable après une revascularisation cardiaque, la progression de l'athérosclérose, l'AVC, la récurrence aux AVC chez un patient ayant des antécédents d'AVC, la récurrence après un accident vasculaire cérébral ischémique, les symptômes du trouble de la conscience ou nerveux causés par un incident cérébro-vasculaire, les engorgements cérébraux, les attaques cérébrales ischémiques transitoires, l'hémorragie sous-arachnoïdienne, la thrombose cérébrale, l'embolie cérébrale, l'infarctus lacunaire, la syncope, les ulcères, la douleur et cryesthesia associé à des artérioscléroses oblitérantes, ou des incidents thrombotiques et/ou athérothrombotiques se produisant lors d'infarctus du myocarde, l'AVC, les incidents ischémiques cérébro-vasculaire, les maladies artérielles périphériques, le syndrome coronarien aigu et syndrome coronarien aigu sans sus-décalage du segment ST (angine de poitrine dite « instable » ou infarctus du myocarde sans onde Q).

7. La composition pour utiliser dans la prévention ou le traitement selon une quelconque des revendications 1 à 6, comprenant du sulfate de clopidogrel et de l'icosapentate d'éthyle comme ingrédients actifs.

8. La composition pour utiliser dans la prévention ou le traitement selon une quelconque des revendications 1 à 7, dans laquelle le rapport de dose entre le sulfate de clopidogrel et l'icosapentate d'éthyle est de 1:10 à 1:80.

9. La composition pour utiliser dans la prévention ou le traitement selon une quelconque des revendications 1 à 8, qui est une formulation composite de sulfate de clopidogrel et d'icosapentate d'éthyle.

10. La composition pour utiliser dans la prévention ou le traitement selon une quelconque des revendications 1 à 9, dans laquelle ladite composition est appliquée comme un inhibiteur de l'agrégation plaquettaire.

11. Utilisation d'au moins un sélectionné parmi le groupe consistant en clopidogrel et ces sels pharmaceutiquement acceptable et au moins un sélectionné parmi le groupe consistant en l'acide icosapentaenoïque aussi bien que ces sels et esters pharmaceutiquement acceptables pour fabriquer une composition pour la prévention ou le traitement d'une maladie ou d'un état associé à un thrombus ou à un embolus d'un sujet souffrant d'une maladie ou d'un état avec des dommages cellulaires de l'endothélium vasculaire à appliquer conjointement, dans laquelle l'application conjointe inclut l'administration d'une formulation contenant les deux ingrédients actifs et l'administration simultanée ou séparée d'un certain laps de temps des deux ingrédients actifs comme formulations distinctes.

12. Au moins un sélectionné parmi le groupe consistant en l'acide icosapentaenoïque aussi bien que ces sels et esters pharmaceutiquement acceptables pour utiliser dans la prévention ou le traitement de la maladie associé à un thrombus ou à un embolus à appliquer en combinaison avec au moins un sélectionné parmi le groupe consistant en clopidogrel et ces sels pharmaceutiquement acceptable de façon simultanée ou séparément avec un certain laps de temps.

13. Au moins un sélectionné parmi le groupe consistant en clopidogrel et ces sels pharmaceutiquement acceptables pour utiliser dans la prévention ou le traitement de la maladie associé à un thrombus ou à un embolus à appliquer en combinaison avec au moins un sélectionné parmi le groupe consistant en l'acide icosapentaenoïque aussi bien que ces sels et esters pharmaceutiquement acceptables de façon simultanée ou séparément avec un certain laps de temps.

## FIG.1

☐ CONTROL GROUP(N=8)
▨ EPA-E GROUP(N=9)
▨ CLOPIDOGREL GROUP(N=9)
■ COMBINATION GROUP(N=9)

\* *p* < 0.05
\*\* *p* < 0.01

## FIG.2

COLLAGEN 6 μg/mL
☐ CONTROL GROUP(N=8)
▨ EPA-E GROUP(N=9)
▨ CLOPIDOGREL GROUP(N=9)
■ COMBINATION GROUP(N=9)

\* *p* < 0.05
\*\* *p* < 0.01

## FIG.3(A)

CONTROL GROUP(N=8)
EPA-E GROUP(N=9)
CLOPIDOGREL GROUP(N=9)
COMBINATION GROUP(N=9)

## FIG.3(B)

CONTROL GROUP(N=8)
EPA-E GROUP(N=9)
CLOPIDOGREL GROUP(N=9)
COMBINATION GROUP(N=9)

# FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 10099046 A
- JP 8157362 A
- JP 8333243 A
- JP 2005021124 A

### Non-patent literature cited in the description

- **YANAGISAWA et al.** *nat,* 1992, vol. 26, 3193-3198
- **YUKIO OZAKI.** *Saishin Igaku (current medicine),* vol. 55 (2), 41-51
- **IKURO MARUYAMA.** *Kekkan To Naihi (blood vessels and endothelia),* 1999, vol. 9 (6), 136-140
- Drug Interview Form of the antiplatelet agent. June 2006, 43, 44
- **ELG et al.** *Thromb. Res.,* 2001, vol. 101 (3), 159-170